# EUROPÄISCHE PATENTANMELDUNG

(11) **EP 4 649 910 A1**
(43) Veröffentlichungstag der Anmeldung: **19.11.2025**
(21) Anmeldenummer: 24175424.1
(22) Anmeldetag: 13.05.2024
(51) Int. Cl.: A61B 34/20

(54) **MEDIZINISCHES, INSBESONDERE CHIRURGISCHES, TISCH-STEUERGERÄT MIT EINER BILDGEBUNGSVORRICHTUNG**

(71) Anmelder: W & H Dentalwerk Bürmoos GmbH, 5111 Bürmoos (AT)
(72) Erfinder: RUSCH, Stefan, 5120 St. Pantaleon (AT); WEBERSBERGER, Thomas, 5142 Eggelsberg (AT); SCHMIED, Isabella, 5274 Burgkirchen (AT); BRANDSTÄTTER, Andreas, 5120 St. Pantaleon (AT); LANG, Thomas, 5421 Adnet (AT)
(74) Vertreter: Benda, Ralf

(57) **Zusammenfassung**

Medizinisches Tisch-Steuergerät (1), umfassend: ein Außengehäuse (2), eine Anschlussvorrichtung (3) an ein mit einer Hand haltbares, medizinisches Behandlungsgerät (4), eine Steuervorrichtung (5) zum Steuern des Tisch-Steuergeräts (1) und/ oder des medizinischen Behandlungsgeräts (4), eine am Außengehäuse (2) angeordnete Fördervorrichtung (6) zum Fördern einer Behandlungs- und/ oder Kühlflüssigkeit, eine Mensch-Maschine-Schnittstelle (7, 8) zum Bedienen des Tisch-Steuergeräts (1) und/ oder des medizinischen Behandlungsgeräts (4) und eine Bildgebungsvorrichtung (9), die Bilddaten von Materialien und/ oder Gegenständen (10) und/ oder von Identifizierungscodes (11) dieser Materialien oder Gegenstände (10), die bei einer medizinischen Behandlung verwendet werden, bei der das Tisch-Steuergerät (1) eingesetzt wird, erzeugt.

Des Weiteren ist ein entsprechendes Verfahren zur Registrierung von Materialien und/ oder Gegenständen (10), die bei einer medizinischen Behandlung, bei der das medizinische, insbesondere chirurgische, Tisch-Steuergeräts (1) eingesetzt wird, verwendet werden, beschrieben.

## Beschreibung

Die vorliegende Erfindung betrifft ein medizinisches, insbesondere chirurgisches, Tisch-Steuergerät mit einer Bildgebungsvorrichtung.

Derartige Tisch-Steuergerät, landläufig auch häufig als Table-Top-Steuergerät bezeichnet, sind sowohl in Krankenhäusern, Kliniken als auch in Arztpraxen weit verbreitet. Sie werden zum Beispiel für rein dentale Anwendungen, wie dem Einsetzen dentaler Implantate oder endodontischer Behandlungen, als auch für orthopädische Eingriffe, beispielsweise im Bereich der Mund-Kiefer-Gesichtschirurgie, der Hals-Nasen-Ohrenchirurgie oder der Hand- und Fußchirurgie eingesetzt.

Der Dokumentation derartiger mit einem Tisch-Steuergerät durchgeführten medizinischer, insbesondere chirurgischer, Behandlungen kommt eine immer noch weiter zunehmende Bedeutung zu. Ein Teil dieser Dokumentation betrifft die Nachverfolgung und/ oder Registrierung der während der Behandlung verwendeten Materialien und Gegenstände, die aktuell oftmals noch zumindest teilweise händisch erfolgt. So notiert ein Assistent, eine Assistentin während der Behandlung Identifizierungscodes der verwendeten Materialien und Gegenstände und/ oder sammelt Verpackungen der verwendeten Materialien und Gegenstände mit den Identifizierungscodes und gibt diese später manuell in einen Computer ein. Alternativ werden teilweise auch Fotos der Identifizierungscodes der verwendeten Materialien und Gegenstände und/ oder Verpackungen mit mobilen Endgeräten angefertigt und diese Fotos anschließend an eine Datenverarbeitungsvorrichtung zur weiteren Verarbeitung übertragen.

Der vorliegenden Erfindung liegt die Aufgabe zugrunde, die Nachverfolgung und/ oder Registrierung der während der Behandlung, bei der ein medizinisches, insbesondere chirurgisches, Tisch-Steuergerät eingesetzt wird, verwendeten Materialien und/ oder Gegenstände für die Anwender zu erleichtern.

Diese Aufgabe wird gemäß der vorliegenden Erfindung durch ein medizinisches, insbesondere chirurgisches, Tisch-Steuergerät mit den Merkmalen des Anspruchs 1 und durch ein Verfahren zur Registrierung von Materialien und/ oder Gegenständen nach Anspruch 15 gelöst. Besonders vorteilhafte Ausführungsformen sind in den Unteransprüchen angeführt.

Das erfindungsgemäße medizinische, insbesondere chirurgische, Tisch-Steuergerät umfasst ein Außengehäuse, welches das Tisch-Steuergerät zur Umgebung abgrenzt, eine am Außengehäuse vorgesehene Anschlussvorrichtung an ein mit einer Hand haltbares, medizinisches Behandlungsgerät, eine im Außengehäuse angeordnete Steuervorrichtung zum Steuern des Tisch-Steuergeräts und/ oder des mit dem Tisch-Steuergerät verbundenen medizinischen Behandlungsgeräts, eine im oder am Außengehäuse angeordnete Fördervorrichtung zum Fördern einer Behandlungs- und/ oder Kühlflüssigkeit, eine Mensch-Maschine-Schnittstelle zum Bedienen des Tisch-Steuergeräts und/ oder des mit dem Tisch-Steuergerät verbundenen medizinischen Behandlungsgeräts und vorzugsweise eine grafische Anzeigevorrichtung zum Anzeigen von Daten des Tisch-Steuergeräts und/ oder des mit dem Tisch-Steuergerät verbundenen medizinischen Behandlungsgeräts. Das medizinische, insbesondere chirurgische, Tisch-Steuergerät weist des Weiteren eine Bildgebungsvorrichtung auf, die Bilddaten von Materialien und/ oder Gegenständen und/ oder von Identifizierungscodes dieser Materialien oder Gegenstände erzeugt, die bei einer medizinischen Behandlung, bei der das Tisch-Steuergerät eingesetzt wird, verwendet werden.

Das erfindungsgemäße Verfahren zur Registrierung von Materialien und/ oder Gegenständen, die bei einer medizinischen Behandlung, bei der ein medizinisches, insbesondere chirurgisches, Tisch-Steuergerät eingesetzt wird, verwendet werden, ist dadurch definiert, dass ein medizinisches, insbesondere chirurgisches, Tisch-Steuergerät mit einer Bildgebungsvorrichtung, vorzugsweise ein medizinisches, insbesondere chirurgisches, Tisch-Steuergerät wie es in dem gegenständlichen Dokument beschrieben ist, zur Verfügung gestellt wird, und dass Bilddaten der Materialien und/ oder Gegenständen, die bei der medizinischen Behandlung verwendet werden, und/ oder von Identifizierungscodes dieser Materialien oder Gegenstände von der Bildgebungsvorrichtung des Tisch-Steuergeräts erzeugt werden.

Ein Tisch-Steuergerät mit einer Bildgebungsvorrichtung, die Bilddaten von Materialien und/ oder Gegenständen, die bei einer medizinischen Behandlung unter Einsatz des Tisch-Steuergeräts verwendet werden, und/ oder deren Identifizierungscodes erzeugt, und ein entsprechendes Verfahren zur Registrierung dieser Materialien und/ oder Gegenstände erleichtern die Registrierung der Identifizierungscodes, Materialien oder Gegenstände erheblich. So entfällt zum Beispiel das im Vorstehenden beschriebene Aufschreiben oder Fotografieren der Identifizierungscodes, Materialien und/ oder Gegenstände und das spätere manuelle Übertragen dieser Daten. Besonders vorteilhaft ermöglicht das Vorsehen der Bildgebungsvorrichtung die Registrierung der Materialien, Gegenstände und/ oder Identifizierungscodes während der Behandlung und insbesondere in der Reihenfolge der Verwendung der Materialien und Gegenstände. Alternativ oder ergänzend ist es natürlich auch möglich nach Abschluss einer Behandlung die Materialien, Gegenstände und/ oder (aufbewahrten) Identifizierungscodes mit der Bildgebungsvorrichtung zu registrieren. Es werden somit sowohl der zeitliche Aufwand als auch der Arbeitsaufwand für die Registrierung der verwendeten Materialien, Gegenstände und/ oder deren Identifizierungscodes merklich reduziert.

Die Bildgebungsvorrichtung ist vorzugsweise als digitale Bildgebungsvorrichtung ausgebildet, insbesondere mit einer digitalen Kamera. Die digitale Bildgebungsvorrichtung oder Kamera umfasst vorzugsweise einen digitalen, lichtempfindlichen Bildsensor, beispielsweise ein CCD-Sensor oder ein CMOS-Sensor. Die Kamera, insbesondere die Kameraoptik und/ oder der Bildsensor, weist vorzugsweise eine feststehende oder unveränderliche Brennweite auf. Alternativ weist die Kamera, insbesondere die Kameraoptik und/ oder der Bildsensor, eine variable Brennweite auf. Die Bildgebungsvorrichtung umfasst vorzugsweise eine Optik oder zumindest ein optisches Element, insbesondere zumindest eine Linse, welche die von den verwendeten Materialien und/ oder Gegenständen, insbesondere deren Identifizierungscodes, einfallende Strahlung auf dem Weg zu dem lichtempfindlichen Sensor passiert. Die Bildgebungsvorrichtung umfasst des Weiteren ein Bilddatenverarbeitungssystem, insbesondere einen Prozessor mit Bildverarbeitungssoftware, der die erfassten Bilddaten verarbeitet und/ oder eine Zeichenerkennung durchführt, so wie dies im Nachfolgenden beschrieben ist. Das Bilddatenverarbeitungssystem bzw. der Prozessor kann dabei entweder als Teil der im Außengehäuse des Tisch-Steuergeräts angeordnete Steuervorrichtung ausgebildet sein oder eine eigenständige, von der Steuervorrichtung getrennte, im Außengehäuse angeordnete Komponente sein. Ist die Bildgebungsvorrichtung in einem separaten Bildgebungsmodul vorgesehen, so kann das Bilddatenverarbeitungssystem bzw. der Prozessor in dem Bildgebungsmodul oder außerhalb davon, zum Beispiel im Außengehäuse des Tisch-Steuergeräts, angeordnet sein. Das Bilddatenverarbeitungssystem bzw. der Prozessor kann alternativ auch entfernt von dem Tisch-Steuergerät oder dem Bildgebungsmodul in einer eigenständigen oder externen Datenverarbeitungsvorrichtung, zum Beispiel einem PC oder in der Cloud, vorgesehen sein.

Die Bildgebungsvorrichtung oder zumindest Teile davon, insbesondere die Kamera und/ oder der Bildsensor, ist/ sind vorzugsweise im Außengehäuse des Tisch-Steuergeräts angeordnet. Damit ist in vorteilhafter Weise ein kompaktes Tisch-Steuergerät ohne zusätzliche externe Komponenten gebildet. Auch ist die Bildgebungsvorrichtung damit vor externen Einflüssen wie Behandlungsflüssigkeiten oder Reinigungsmitteln geschützt. Das Außengehäuse weist vorzugsweise einen Strahlungsdurchlass auf, durch welchen die Bildgebungsvorrichtung, insbesondere der Bildsensor, Strahlung empfängt, vorzugsweise von den verwendeten Materialien, Gegenständen und/ oder deren Identifizierungscodes. Der Strahlungsdurchlass umfasst zum Beispiel eine Bildgebungs-Öffnung oder Apertur oder einen strahlungsdurchlässigen, zum Beispiel ungefärbten, Abschnitt des Außengehäuses.

Der Strahlungsdurchlass ist vorzugsweise an einer Frontseite des Außengehäuses vorgesehen. Die Frontseite ist insbesondere jene Seite des Außengehäuses, an der weitere Bedienelemente des Tisch-Steuergeräts, zum Beispiel zumindest Teile der Mensch-Maschine-Schnittstelle, und/ oder die grafische Anzeigevorrichtung und/ oder die Anschlussvorrichtung an ein mit einer Hand haltbares, medizinisches Behandlungsgerät vorgesehen sind. Die Frontseite ist für den Anwender somit frei zugänglich und gut erreichbar, so dass das Vorsehen des Strahlungsdurchlasses an der Frontseite auch die Erfassung der Bilddaten oder Bildaufnahme in vorteilhafter Weise vereinfacht. Alternativ ist es auch denkbar, den Strahlungsdurchlass und gegebenenfalls die Bildgebungsvorrichtung an einer anderen Fläche des Außengehäuses vorzusehen, zum Beispiel an der Oberseite oder an Seitenwänden des Außengehäuses.

Alternativ ist ein Bildgebungsmodul vorgesehen, in dem die Bildgebungsvorrichtung oder zumindest ein Teil davon angeordnet ist, wobei das Bildgebungsmodul außerhalb des Außengehäuses des Tisch-Steuergeräts und/ oder entfernt und/ oder getrennt von dem Tisch-Steuergerät ausgebildet ist. Das Bildgebungsmodul ist somit ein eigenständiges, separates Element, das insbesondere in vorteilhafter Weise zum Nachrüsten von bereits am Markt oder in Verwendung befindlicher Tisch-Steuergeräte verwendet werden kann. Im Inneren des Bildgebungsmoduls ist zumindest die digitale Kamera und/ oder der digitale Bildsensor der Bildgebungsvorrichtung angeordnet. Vorzugsweise können auch weitere Bauteile der Bildgebungsvorrichtung, zum Beispiel ein optisches Element, insbesondere zumindest eine Linse, welche die von den verwendeten Materialien und/ oder Gegenständen, insbesondere deren Identifizierungscodes, einfallende Strahlung zu dem lichtempfindlichen Sensor leitet, und/ oder ein Bilddatenverarbeitungssystem, insbesondere einen Prozessor mit Bildverarbeitungssoftware, in oder an dem Bildgebungsmodul vorgesehen sein, so dass das Bildgebungsmodul in vorteilhafter Weise eine komplette Nachrüsteinheit bildet. Das Bildgebungsmodul umfasst vorzugsweise auch eine Kommunikationsvorrichtung zur Übertragung der von der Bildgebungsvorrichtung erfassten oder zur Verfügung gestellten Bilddaten an eine externe Datenverarbeitungsvorrichtung und/ oder Steuervorrichtung.

Ein Modulgehäuse grenzt das Bildgebungsmodul zur Umgebung ab. Das Modulgehäuse weist vorzugsweise einen Strahlungsdurchlass auf, durch welche die Bildgebungsvorrichtung, insbesondere der Bildsensor, Strahlung empfängt, vorzugsweise von den verwendeten Materialien, Gegenständen und/ oder deren Identifizierungscodes. Der Strahlungsdurchlass umfasst zum Beispiel eine Bildgebungs-Öffnung oder Apertur oder einen strahlungsdurchlässigen, zum Beispiel ungefärbten, Abschnitt des Modulgehäuses. Die empfangene Strahlung umfasst vorzugsweise für den Menschen sichtbares Licht, insbesondere Teilspektren davon, und/ oder Infrarotstrahlung.

Der Strahlungsdurchlass des Tisch-Steuergeräts oder des Bildgebungsmoduls ist vorzugsweise kreisförmig oder elliptisch und hat in etwa einen Durchmesser von 0,5 - 3,0 cm. Besonders bevorzugt ist in dem oder anschließend an den Strahlungsdurchlass ein optisches Element der Bildgebungsvorrichtung vorgesehen, zum Beispiel eine optische Linse oder ein optischer Filter, der die empfangene Strahlung für die Bildgebungsvorrichtung filtert. Das Vorsehen eines optischen Elements in dem oder anschließend an den Strahlungsdurchlass ermöglicht in vorteilhafter Weise eine platzsparende Positionierung des optischen Elements.

Das Tisch-Steuergerät oder die Bildgebungsvorrichtung weist besonders bevorzugt eine Strahlungsquelle auf, die Strahlung mit einem begrenzten Spektralbereich oder Teilbereich des für ein menschliches Auge sichtbaren Lichtspektrums und/ oder Infrarotstrahlung emittiert, zur Beleuchtung der Identifizierungscodes, der Materialien und/ oder Gegenstände während der Erfassung der Bilddaten. Der begrenzten Spektralbereich umfasst ein Teilspektrum innerhalb des für ein menschliches Auge sichtbaren Lichtspektrums. Die Strahlungsquelle ist vorzugsweise in oder an dem Außengehäuse des Tisch-Steuergeräts oder dem Modulgehäuse des Bildgebungsmoduls angeordnet, wodurch in vorteilhafter Weise ein kompaktes Tisch-Steuergerät oder Bildgebungsmodul ohne zusätzliche externe Komponenten gebildet ist. Auch ist die Strahlungsquelle damit vor externen Einflüssen wie Behandlungsflüssigkeiten oder Reinigungsmitteln geschützt. Das Beleuchten der Identifizierungscodes, der Materialien und/ oder Gegenstände während der Erfassung der Bilddaten durch die Bildgebungsvorrichtung erhöht in vorteilhafter Weise die Qualität der Bilddaten.

Die von der Strahlungsquelle emittierte und gegebenenfalls anschließend mit einem optischen Element geformte Infrarotstrahlung und/ oder sichtbare Strahlung mit einem begrenzten Spektralbereich umfasst zum Beispiel monochromatische Strahlung oder monochromatisches Licht, Strahlung nur einer Spektralfarbe, mit wenigen Wellenlängen oder mit einer einzigen Wellenlänge. Das für den Menschen sichtbare Licht mit einem begrenzten Spektralbereich umfasst somit kein Weißlicht. Die Beleuchtung der Identifizierungscodes, der Materialien und/ oder Gegenstände während der Erfassung der Bilddaten mit Strahlung mit einem begrenzten Spektralbereich erleichtert in vorteilhafter Weise die Bildaufnahme durch die Bildgebungsvorrichtung und verbessert die Qualität und Auswertbarkeit der erfassten Bilddaten, da negative optische Einflüsse wie Reflexion oder Blendung durch andere Strahlungsquellen, vor allem von Weißlichtquellen wie zum Beispiel der Raumbeleuchtung, von OP-Leuchten, Stirnlampen und/ oder des natürlichen Tageslichts, verringert werden.

Die Strahlungsquelle ist insbesondere derart angeordnet oder positioniert, dass sie Strahlung in Richtung oder in einen Bildaufnahmebereich emittiert, in den die Materialien, Gegenständen und/ oder Identifizierungscodes eingebracht werden (müssen), damit eine Bildaufnahme durch die Bildgebungsvorrichtung erfolgt oder möglich ist. Der Bildaufnahmebereich ist ein Raumvolumen, das in Bezug auf die Bildgebungsvorrichtung, insbesondere den Bildsensor, derart angeordnet, dass die von der Bildgebungsvorrichtung erzeugten Bilddaten für das Bilddatenverarbeitungssystem zum Erfassen von Informationen auswertbar sind und/ oder eine Zeichenerkennung ermöglichen. Dazu müssen die Bilddaten zum Beispiel einen entsprechenden Kontrast, eine entsprechende Größe, Bildschärfe oder Auflösung haben. Der Bildaufnahmebereich, insbesondere die Entfernung des Bildaufnahmebereichs von dem Bildsensor und/ oder dem Strahlungsdurchlass, ist somit auf die Brennweite der Bildgebungsvorrichtung abgestimmt, die vorzugsweise unveränderlich ist.

Das Außengehäuse des Tisch-Steuergeräts oder das Modulgehäuse des Bildgebungsmoduls weist vorzugsweise einen Beleuchtungsaustritt auf, durch welche Strahlung der im Inneren des Tisch-Steuergeräts oder des Modulgehäuses angeordneten Strahlungsquelle in die Umgebung, insbesondere in den Bildaufnahmebereich, emittierbar ist. Der Beleuchtungsaustritt ist vorzugsweise an derselben Seite oder Fläche des Außengehäuses oder des Modulgehäuses wie der Strahlungsdurchlass vorgesehen, insbesondere an der Frontseite des Außengehäuses. Damit ist es in vorteilhafter Weise möglich, die Strahlungsquelle und die Bildgebungsvorrichtung, insbesondere den Bildsensor, optimal relativ zueinander und insbesondere auch zum Bildaufnahmebereich zu positionieren, so dass Bilddaten mit guter Qualität erzeugbar sind.

Der Beleuchtungsaustritt umfasst zum Beispiel eine Öffnung im Außengehäuse oder Modulgehäuse oder einen strahlungsdurchlässigen, zum Beispiel ungefärbten, Abschnitt des Außengehäuses, insbesondere der Frontseite, so wie dies im Vorstehenden in Bezug auf den Strahlungsdurchlass beschrieben ist.

Die Strahlungsquelle zur Abgabe von Strahlung mit einem begrenzten Spektralbereich und/ oder Infrarotstrahlung umfasst zum Beispiel ein optisches Halbleiterelement, insbesondere eine Leuchtdiode. Damit wird in vorteilhafter Weise Strahlung mit einem begrenzten Spektralbereich erzeugt, ohne dass dafür weitere Bauteile, wie Filter etc. notwendig sind. Selbstverständlich sind jedoch auch andere Arten von Strahlungsquellen möglich, zum Beispiel Glühbirnen oder Leuchtstofflampen. Um den Spektralbereich der von der Strahlungsquelle abgestrahlten Strahlung, auch eines optisches Halbleiterelements, zu verringern, zum Beispiel auf nur wenige oder eine einzige Wellenlänge, kann die Strahlungsquelle ein entsprechendes optisches Element, zum Beispiel einen optischen Filter, ein Beugungsgitter und/ oder ein Prisma, aufweisen. Das optische Element ist insbesondere im Strahlengang der abgestrahlten Strahlung zwischen der Strahlungsquelle und dem Beleuchtungsaustritt angeordnet, so dass die abgestrahlte Strahlung vor dem Austritt aus dem Beleuchtungsaustritt das optische Element passiert.

Alternativ oder zusätzlich ist die Strahlungsquelle zur Beleuchtung der Identifizierungscodes, der Materialien und/ oder Gegenstände während der Erfassung der Bilddaten durch die grafische Anzeigevorrichtung des Tisch-Steuergeräts gebildet. Die Verwendung der grafischen Anzeigevorrichtung als Strahlungsquelle hat den Vorteil, dass keine zusätzliche Strahlungsquelle an dem Tisch-Steuergerät vorzusehen ist. Vorzugsweise ist die grafische Anzeigevorrichtung derart angeordnet oder positioniert, dass sie Strahlung in Richtung oder in den im Vorstehenden beschriebenen Bildaufnahmebereich emittiert. Vorzugsweise sind die grafische Anzeigevorrichtung und der Bildaufnahmebereich geradlinig zueinander angeordnet. Vorzugsweise ist die optische Achse der Bildgebungsvorrichtung, insbesondere der Kamera oder des Bildsensors, schräg oder gewinkelt zur grafischen Anzeigevorrichtung, insbesondere deren Hauptabstrahlrichtung, angeordnet, bevorzugt derart, dass die optische Achse den Bildaufnahmebereich und/ oder die Abstrahlrichtung der von der grafischen Anzeigevorrichtung abgestrahlten Strahlung oder Hauptabstrahlrichtung schneidet. Damit sind in vorteilhafter Weise Bilddaten mit guter Qualität erzeugbar.

Besonders bevorzugt strahlt die Strahlungsquelle grünes Licht, i.e. Strahlung mit einer Wellenlänge im Bereich von etwa 500 nm - 570 nm, zur Beleuchtung der Identifizierungscodes, der Materialien und/ oder Gegenstände während der Erfassung der Bilddaten ab. Besonders bevorzugt emittiert die Strahlungsquelle grüne Strahlung mit einer einzigen Wellenlänge von 525 nm oder mit einem Emissionsspektrum mit einem Peak bei 525 nm. Bevorzugt wird als Strahlungsquelle ein grüne Strahlung emittierendes optisches Halbleiterelement (LED) verwendet, das zum Beispiel Galliumnitrid oder Aluminiumgalliumphosphid aufweist. Wie im Vorstehenden bereits beschrieben, erhöht die Beleuchtung der Identifizierungscodes, der Materialien und/ oder Gegenstände durch die Strahlungsquelle während der Erfassung der Bilddaten in vorteilhafter Weise die Qualität der Bilddaten, indem negative optische Einflüsse wie Reflexion oder Blendung durch andere Strahlungsquellen, vor allem von Weißlichtquellen, verringert werden. In Versuchen wurde festgestellt, dass grünes Licht hierfür am geeignetsten ist, d.h. die Qualität am höchsten und die folgende Verarbeitung der Bilddaten besonders gut ist. Ohne sich auf eine bestimmte Theorie festlegen zu wollen, wird vermutet, dass die höhere Qualität der Bilddaten mit dem geringeren Anteil an grünem Licht (im Vergleich zu zum Beispiel blauem Licht) in dem im medizinischen Umfeld vorkommenden Weißlicht zusammenhängt.

Die Bildgebungsvorrichtung weist bevorzugt einen optischen Filter auf, der die von den Identifizierungscodes, Materialien und/ oder Gegenständen einfallende Strahlung filtert. Damit kann in vorteilhafter Weise die Qualität der erzeugten Bilddaten weiter verbessert werden. Besonders bevorzugt ist der optische Filter im Wesentlichen oder ausschließlich für Wellenlängen durchlässig, die in der von der im Vorstehenden beschriebenen Strahlungsquelle emittierten Strahlung (Infrarotstrahlung und/ oder sichtbares Licht mit einem begrenzten Spektralbereich) enthalten sind, insbesondere für grünes Licht, vorzugsweise mit Wellenlängen im Bereich von etwa 500 nm - 570 nm. Damit ist sichergestellt, dass nur dann Bilddaten (mit auswertbarer Bildinformation) durch die Kamera erzeugbar sind, wenn die Strahlungsquelle zur Beleuchtung der Identifizierungscodes, der Materialien und/ oder Gegenstände Strahlung emittiert. In vorteilhafter Weise wird somit eine unerwünschte Bilderfassung durch die Kamera verhindert, insbesondere wenn die Erfassung der Bilddaten und damit die Emission von Strahlung durch die Strahlungsquelle durch ein Stellelement aktivierbar ist, so wie dies im Folgenden beschrieben ist.

Der optische Filter ist vorzugsweise als Farbfilter ausgebildet, der insbesondere Teile der von ihm empfangenen Strahlung absorbiert. Besonders bevorzugt ist der Filter als Grünfilter ausgebildet, der für grünes Licht, d.h. Wellenlängen von in etwa 500 nm - 570 nm durchlässig, und für alle anderen Wellenlängen nicht durchlässig ist. Besonders bevorzugt umfasst das Tisch-Steuergerät oder das Bildgebungsmodul eine Strahlungsquelle, die grünes Licht emittiert, insbesondere mit einer einzigen Wellenlänge von 525 nm oder mit einem Emissionsspektrum mit einem Peak bei 525 nm, und einen entsprechenden optischen Filter, der ausschließlich für grünes Licht durchlässig ist, insbesondere für grünes Licht mit einer einzigen Wellenlänge von 525 nm oder mit einem Spektrum mit einem Peak bei 525 nm. Mit dieser Konfiguration konnte in Versuchen die höchste Qualität der Bilddaten erzielt werden.

Der optische Filter ist beispielsweise an dem oder anschließend an den Strahlungsdurchlass oder im Strahlengang zwischen dem Strahlungsdurchlass und der Kamera oder dem Bildsensor angeordnet, so dass die von den Materialien, Gegenständen und/ oder Identifizierungscodes empfangene Strahlung vor dem Auftreffen auf dem Bildsensor den optischen Filter passiert.

Die Bildgebungsvorrichtung, die Kamera oder der Bildsensor, insbesondere deren optische Achse, ist vorzugsweise schräg, insbesondere in einem Winkel ungleich 90°, zu der Frontseite des Tisch-Steuergeräts oder des Bildgebungsmoduls angeordnet. Die Bildgebungsvorrichtung, die Kamera oder der Bildsensor sind zum Beispiel seitlich der grafischen Anzeigevorrichtung angeordnet und derart in Richtung der grafischen Anzeigevorrichtung gedreht, dass ihre optische Achse in Richtung der grafischen Anzeigevorrichtung weist. Damit ist die Bildgebungsvorrichtung, die Kamera oder der Bildsensor dem Bildaufnahmebereich zugewandt, wodurch die Qualität der erzeugten Bilddaten verbessert wird.

Die Strahlungsquelle, die Strahlung zum Beleuchten der Materialien, Gegenständen und/ oder Identifizierungscodes emittiert, insbesondere eine Längsachse oder Kegelachse eines abgestrahlten Strahlungskegels der Strahlungsquelle, ist vorzugsweise schräg, insbesondere in einem Winkel ungleich 90°, zu einer Frontseite des Tisch-Steuergeräts oder des Bildgebungsmoduls angeordnet. Die Strahlungsquelle ist zum Beispiel seitlich der grafischen Anzeigevorrichtung angeordnet und derart in Richtung der grafischen Anzeigevorrichtung gedreht, dass der abgestrahlte Strahlungskegel in Richtung der grafischen Anzeigevorrichtung weist. Damit ist die Strahlungsquelle dem Bildaufnahmebereich zugewandt, wodurch die Qualität der erzeugten Bilddaten verbessert wird.

Die Bildgebungsvorrichtung, insbesondere die Kamera, und die Strahlungsquelle sind bevorzugt derart angeordnet, dass das Sichtfeld, insbesondere die optische Achse, der Bildgebungsvorrichtung, und der von der Strahlungsquelle abgestrahlte Strahlungskegel, insbesondere die Kegelachse des Strahlungskegels, einander treffen oder schneiden. Bevorzugt erstrecken sich das Sichtfeld, insbesondere die optische Achse, und der abgestrahlte Strahlungskegel, insbesondere die Kegelachse, in den Bildaufnahmebereich. Besonders bevorzugt schneiden sich das Sichtfeld und der abgestrahlte Strahlungskegel in dem Bildaufnahmebereich. Besonders bevorzugt ist der Drehwinkel, um den die Bildgebungsvorrichtung oder der Bildsensor, insbesondere deren optische Achse, in Richtung der grafischen Anzeigevorrichtung und der Drehwinkel, um den die Strahlungsquelle, insbesondere eine Längsachse oder Kegelachse eines abgestrahlten Strahlungskegels, in Richtung der grafischen Anzeigevorrichtung gedreht sind, identisch in Bezug auf eine die Bildschirmoberfläche der grafischen Anzeigevorrichtung durchsetzende Normale. Durch diese Merkmale sind in vorteilhafter Weise eine optimale Beleuchtung der Identifizierungscodes, Materialien und/ oder Gegenstände und Bilddaten mit hoher optischer Schärfe garantiert.

Vorzugsweise ist im Außengehäuse des Tisch-Steuergeräts oder in dem Bildgebungsmodul ein Bilddatenverarbeitungssystem zur Verarbeitung der von der Bildgebungsvorrichtung erfassten Bilddaten angeordnet. Alternativ ist es auch möglich, dass das Bilddatenverarbeitungssystem außerhalb und/ oder entfernt von dem Tisch-Steuergerät oder dem Bildgebungsmodul vorgesehen ist, zum Beispiel in einer eigenständigen oder externen Datenverarbeitungsvorrichtung, zum Beispiel einem PC oder in der Cloud. In diesem Fall umfassen das Tisch-Steuergerät oder das Bildgebungsmodul eine Kommunikationsvorrichtung zur Übertragung der von der Bildgebungsvorrichtung erfassten Bilddaten an die externe Datenverarbeitungsvorrichtung. Das Vorsehen des Bilddatenverarbeitungssystems in dem Tisch-Steuergerät oder Bildgebungsmodul hat den Vorteil, dass durch die Verarbeitung, insbesondere Auswahl, von Bilddaten, welche die von der Bildgebungsvorrichtung aufgenommenen Identifizierungscodes, Materialien und/ oder Gegenständen besonders gut wiedergeben, eine Reduktion des Datenvolumens erreicht wird (im Vergleich zur Gesamtheit der aufgenommenen Bilddaten), das in Folge vom Tisch-Steuergerät oder Bildgebungsmodul an eine externe Datenverarbeitungsvorrichtung übertragen wird.

Das Bilddatenverarbeitungssystem umfasst insbesondere einen Prozessor mit Bildverarbeitungssoftware. Das Bilddatenverarbeitungssystem ist zum Beispiel für zumindest eine der folgenden Aufgaben vorgesehen, wodurch insbesondere in vorteilhafter Weise eine zuverlässigere Zeichenerkennung in den Bilddaten erreicht wird:
- Umwandlung der von der Bildgebungsvorrichtung erfassten Bilddaten in schwarzweiß Bilddaten oder Graustufen-Bilddaten. Dies ist insbesondere dann vorteilhaft, wenn die Identifizierungscodes, Materialien und/ oder Gegenstände bei der Erfassung durch die Bildgebungsvorrichtung mit Strahlung mit einem Teilbereich des für Menschen sichtbaren Lichtspektrums und/ oder Infrarotstrahlung beleuchtet wurden, so wie dies im Vorstehenden beschrieben ist.
- Korrektur optischer oder perspektivischer Verzerrungen. Dies ist insbesondere von Vorteil, wenn, wie im Vorstehenden beschrieben, die Bildgebungsvorrichtung, insbesondere der Bildsensor, schräg oder gewinkelt zum Bildaufnahmebereich und/ oder zur grafischen Anzeigevorrichtung angeordnet ist.
- Filtern der von der Bildgebungsvorrichtung erfassten Bilddaten, zum Beispiel zum Verändern des Kontrasts oder der Helligkeit der aufgenommenen Bilddaten.
- Korrektur der Farbtemperatur, insbesondere Weißabgleich.
- Korrektur der optischen Schärfe und/ oder des optischen Rauschens der von der Bildgebungsvorrichtung erfassten Bilddaten.
- Optische (maschinelle) Zeichenerkennung von Codes und/ oder Symbolen und/ oder alphanumerischen Zeichen (OCR - Optical Character Recognition), die in den von der Bildgebungsvorrichtung erfassten Bilddaten enthalten sind. Die Zeichenerkennung ist insbesondere vorteilhaft, wenn die Materialien und/ oder Gegenstände über darauf und/ oder auf deren Verpackungen applizierte Identifizierungscodes, wie zum Beispiel Codes, Symbole und/ oder alphanumerische Zeichen, dokumentiert werden. Die Codes können zum Beispiel einen QR-Code, Strichcode oder Data Matrix Code umfassen.
- Bestimmen und/ oder Ändern der Belichtungszeit der Strahlungsquelle der Bildgebungsvorrichtung.
- Bestimmen und/ oder Ändern des Verstärkungsfaktors zur Verstärkung der von der Bildgebungsvorrichtung, insbesondere dem Kamerasensor, erzeugten Bilddaten.

Die im Vorstehenden genannten und gegebenenfalls weitere Bildverarbeitungsprozesse, die von dem Bilddatenverarbeitungssystem durchführbar sind, sind wohlbekannte, rechnerimplementierte Verfahren zur Bearbeitung digitaler Bilddaten, so dass darauf nicht näher eingegangen wird.

Das Tisch-Steuergerät oder das Bildgebungsmodul umfasst bevorzugt eine Signalvorrichtung, die operativ mit dem Bilddatenverarbeitungssystem verbunden und derart ausgebildet ist, dass, wenn das Bilddatenverarbeitungssystem erkennt, dass während der Erfassung der Identifizierungscodes, Materialien und/ oder Gegenständen zumindest eine optische Eigenschaft, insbesondere der Kontrast, die Bildschärfe und/ oder Auflösung, der erfassten oder zur Verfügung gestellten Bilddaten einen vorbestimmten Grenzwert erreicht und/ oder überschreitet, und/ oder wenn das Bilddatenverarbeitungssystem einen Code und/ oder ein Symbol und/ oder ein alphanumerisches Zeichen in den von der Bildgebungsvorrichtung erfassten und/ oder zur Verfügung gestellten Bilddaten erkennt, ein für den Anwender wahrnehmbares Signal abgibt. Somit weiß der Anwender in vorteilhafter Weise, dass die Bildgebungsvorrichtung Bilddaten generieren konnte, die für das Bilddatenverarbeitungssystem auswertbar und somit in weiterer Folge für die Dokumentation der medizinischen Behandlung verwendbar sind. Die Signalabgabe ist für den Anwender auch ein Hinweis, dass sich der Identifizierungscode, die Materialien und/ oder Gegenständen im Bildaufnahmebereich befinden, d.h. in der in Bezug auf die (feststehende) Brennweite der Bildgebungsvorrichtung notwendigen Entfernung zu dem Bildsensor zur Erzeugung von Bilddaten mit auswertbarer Information.

Das Signal ist insbesondere ein akustisches Signal, die Signalvorrichtung umfasst dem entsprechend einen akustischen, vorzugsweise elektronischen, Signalgeber, der insbesondere in oder an dem Tisch-Steuergerät oder Bildgebungsmodul vorgesehen ist. Alternativ ist das Signal ein visuelles Signal, die Signalvorrichtung umfasst dem entsprechend beispielsweise einen visuellen Signalgeber, zum Beispiel ein optisches Halbleiterelement oder die grafische Anzeigevorrichtung des Tisch-Steuergeräts.

Wie im Vorstehenden bereits erwähnt umfasst das Tisch-Steuergerät oder das Bildgebungsmodul bevorzugt eine Kommunikationsvorrichtung zur Übertragung der von der Bildgebungsvorrichtung erfassten, gegebenenfalls von dem Bilddatenverarbeitungssystem bearbeiteten, Bilddaten an eine separate oder externe Datenverarbeitungsvorrichtung. Die Kommunikationsvorrichtung kann drahtlos und/ oder drahtgebunden sein. Damit besteht in vorteilhafter Weise die Möglichkeit, die von der Bildgebungsvorrichtung generierten Bilddaten oder Bilder in der externen Datenverarbeitungsvorrichtung zu bearbeiten, speichern, kommentieren und/ oder mit weiteren Daten, zum Beispiel Patientendaten, zu verbinden.

Das im Vorstehenden schon beschriebene Verfahren zur Registrierung von Materialien und/ oder Gegenständen, die bei einer medizinischen Behandlung, bei der ein medizinisches, insbesondere chirurgisches, Tisch-Steuergerät eingesetzt wird, verwendet werden, ist insbesondere dadurch definiert, dass ein Anwender einen Identifizierungscode, Materialien und/ oder einen Gegenstand, der/ die bei der medizinischen Behandlung verwendet werden oder wurden, in das Sichtfeld der Bildgebungsvorrichtung, insbesondere - bei unveränderbarer Brennweite der Bildgebungsvorrichtung - in den Bildaufnahmebereich hält, so dass die Bildgebungsvorrichtung Bilddaten des Identifizierungscodes, der Materialien und/ oder des Gegenstands generieren kann.

Vorzugsweise aktiviert der Anwender vorher die Bildgebungsvorrichtung und gegebenenfalls die Strahlungsquelle und/ oder startet die Erfassung der Bilddaten oder Bildaufnahme durch die Bildgebungsvorrichtung. Dazu ist an dem Tisch-Steuergerät oder Bildgebungsmodul ein Stellelement vorgesehen. Das Stellelement kann zum Beispiel einen Druckknopf, insbesondere als Teil der Mensch-Maschine-Schnittstelle, auf dem Tisch-Steuergerät oder Bildgebungsmodul umfassen oder durch die als Touch-Screen ausgebildete grafische Anzeigevorrichtung realisiert sein. Alternativ kann das Stellelement als Sprachsteuerung ausgebildet sein. Durch die Betätigung des Stellelements erzeugt der Anwender ein Stellsignal, welches die Bildgebungsvorrichtung und gegebenenfalls die Strahlungsquelle und das Bilddatenverarbeitungssystem aktiviert, so dass die Bildgebungsvorrichtung Bilddaten erzeugt. Das Stellelement ist in vorteilhafter Weise, zum Beispiel durch nochmalige Betätigung, auch dafür vorgesehen, die Bildgebungsvorrichtung und gegebenenfalls die Strahlungsquelle zu deaktivieren und/ oder die Bilddatenerfassung durch die Bildgebungsvorrichtung zu beenden. Durch das Vorsehen des Stellelements wird in vorteilhafter Weise sichergestellt, dass die Bildgebungsvorrichtung nur zu einem gewünschten Zeitpunkt Bilddaten erzeugt und zum Beispiel während einer medizinischen Behandlung keine Bildaufnahmen erfolgen.

Vorzugsweise umfasst das Verfahren zur Registrierung des Weiteren, dass die Signalvorrichtung ein Signal abgibt, zum Beispiel einen Piepton, das dem Anwender anzeigt, dass ein Identifizierungscode, Material und/ oder Gegenstand derart durch die Bildgebungsvorrichtung aufgenommen wurden, dass die erzeugten Bilddaten für das Bilddatenverarbeitungssystem auswertbar und somit in weiterer Folge für die Dokumentation der medizinischen Behandlung verwendbar sind, so wie dies im Vorstehenden bereits beschrieben ist. Nach der Abgabe des Signals durch die Signalvorrichtung kann der Anwender, wenn vorhanden, einen weiteren Identifizierungscode, Gegenstand und/ oder ein weiteres Material nehmen und in das Sichtfeld und/ oder den Bildaufnahmebereich der Bildgebungsvorrichtung halten.

Vorzugsweise umfassen die in dem Verfahren zur Registrierung und/ oder durch die Bildgebungsvorrichtung aufgenommenen Materialien und/ oder Gegenstände zumindest eines der folgenden Elemente und / oder zumindest einen Identifizierungscode der folgenden Elemente:
- Materialien und/ oder Gegenstände, die im oder am Patienten, der mit dem Tisch-Steuergerät behandelt wird, verbleiben, zum Beispiel Prothesen, Implantate, Knochenersatzmaterialien, Füllstoffe und Ähnliches;
- Verbrauchs- und Behandlungsmaterial, das während der Behandlung des Patienten mit dem Tisch-Steuergerät verwendet wird, zum Beispiel chirurgisches Nadeln, Spritzen, Desinfektionsmittel, Nahtmaterial, Anästhetika, Medikamente, Spülflüssigkeiten, sterile Tücher und Ähnliches;
- Ein medizinisches Diagnose- oder Behandlungsgerät, das während der der Behandlung des Patienten mit dem Tisch-Steuergerät verwendet wird, insbesondere durch das Tisch-Steuergerät gesteuert/angetrieben wird, zum Beispiel ein Hand- oder Winkelstück, ein Antriebsmotor für ein medizinisches Instrument, eine medizinische Kamera und Ähnliches;
- Verpackungen der genannten Materialien oder Gegenstände;
- Etiketten zum Nachweis der Reinheit oder Sterilität der genannten Materialien oder Gegenstände, die zum Beispiel nach der Reinigung, Pflege, Desinfektion und/ oder Sterilisation erstellt werden, um Reinheit oder Sterilität der Materialien oder Gegenstände nachzuweisen.

Das Tisch-Steuergerät ist vorzugsweise als dental-chirurgisches Implantationsgerät zum Implantieren dentaler Implantate ausgebildet. Das Tisch-Steuergerät kann jedoch auch für andere Anwendungen ausgebildet sein, insbesondere für orthopädische Eingriffe, beispielsweise im Bereich der Mund-Kiefer-Gesichtschirurgie, der Hals-Nasen-Ohrenchirurgie oder der Hand- und Fußchirurgie.

Die Erfindung wird nachfolgend anhand bevorzugter Ausführungsbeispiele und Bezug nehmend auf die beigefügten Zeichnungen erläutert. Es zeigt die
Figur 1 ein erstes Ausführungsbeispiel eines medizinischen, insbesondere chirurgischen, Tisch-Steuergeräts mit einer Bildgebungsvorrichtung, die Bilddaten von Materialien und/ oder Gegenständen, die bei einer medizinischen Behandlung, bei der das Tisch-Steuergerät eingesetzt wird, verwendet werden, und/ oder von Identifizierungscodes dieser Materialien oder Gegenstände erzeugt;
Figur 2 die Kamera der Bildgebungsvorrichtung und die Strahlungsquelle im Inneren des Außengehäuses des Tisch-Steuergeräts der Figur 1;
Figur 3 ein zweites Ausführungsbeispiel eines medizinischen, insbesondere chirurgischen, Tisch-Steuergeräts mit einer Bildgebungsvorrichtung, die Bilddaten von Materialien und/ oder Gegenständen, die bei einer medizinischen Behandlung, bei der das Tisch-Steuergerät eingesetzt wird, verwendet werden, und/ oder von Identifizierungscodes dieser Materialien oder Gegenstände erzeugt, wobei die Bildgebungsvorrichtung außerhalb und/ oder entfernt von dem Außengehäuse des Tisch-Steuergeräts vorgesehen ist;
Figur 4 eine schematische Darstellung einer Bilderfassung durch die Bildgebungsvorrichtung der Figur 1 oder 3.

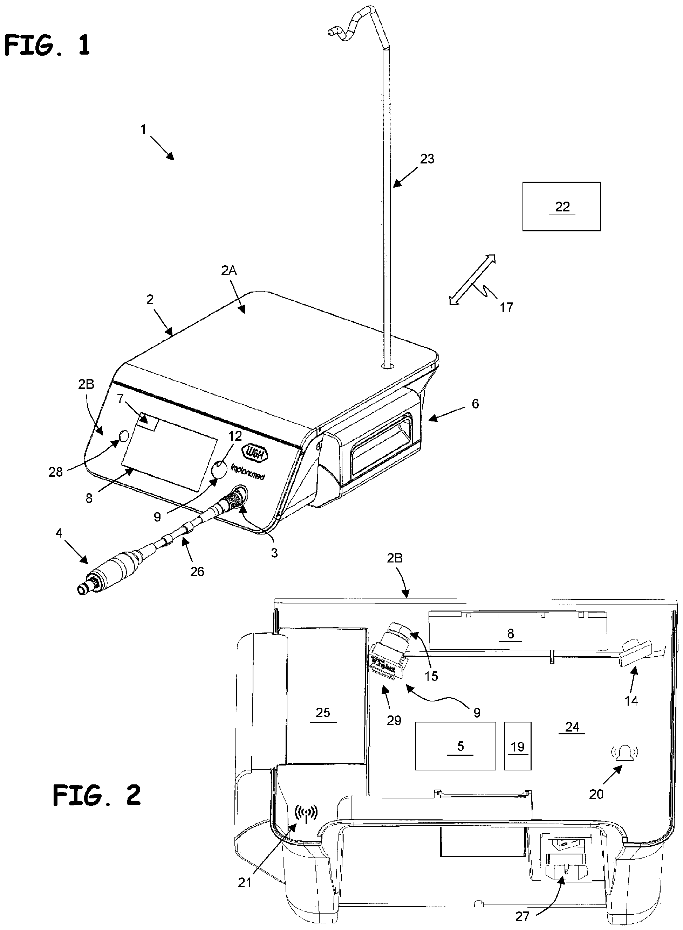

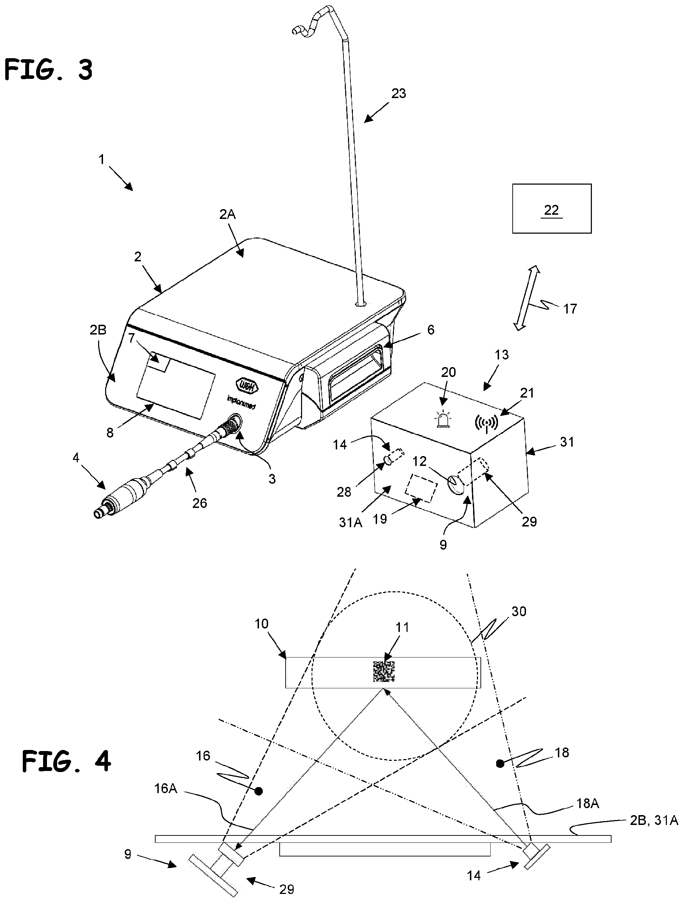

Im Folgenden sind jene Merkmale beschrieben, welche die Tisch-Steuergeräte 1 der Figur 1 und der Figur 3 gemein haben.

Das in den Figuren 1 und 3 dargestellte medizinische, insbesondere chirurgische, Tisch-Steuergerät 1 ist ein kompaktes, handliches Gerät, das derart dimensioniert ist, dass es auf einer Tischplatte, einer Ablagefläche eines Behandlungscarts, an oder auf einer Dentaleinheit oder ähnlichen Oberflächen abstellbar und darauf positioniert für medizinische Behandlungen verwendbar ist. Das Tisch-Steuergerät 1 umfasst ein Außengehäuse 2, welches das Tisch-Steuergerät 1 zur Umgebung abgrenzt. Das Außengehäuse 2 weist eine Oberseite 2A, eine der Oberseite gegenüberliegende Bodenfläche und mehrere die Oberseite 2A und die Bodenfläche verbindende Seitenflächen auf. Eine der Seitenflächen ist als Frontseite 2B ausgebildet, insbesondere ist das jene Seite, an der Bedienelemente des Tisch-Steuergeräts 1, zum Beispiel zumindest Teile einer Mensch-Maschine-Schnittstelle 7, und/ oder eine grafische Anzeigevorrichtung 8 und/ oder eine Anschlussvorrichtung 3 an ein mit einer Hand haltbares, medizinisches Behandlungsgerät 4 vorgesehen sind. An einer der Seitenflächen, vorzugsweise an einer der Frontseite 2B gegenüberliegenden Rückwand, ist/ sind des Weiteren ein Anschluss 27 an eine elektrische Energieversorgung für das Tisch-Steuergerät 1 und gegebenenfalls ein Schalter zum Ein- und Ausschalten des Tisch-Steuergeräts 1 vorgesehen.

An der Oberseite 2A ist eine Öffnung zur Aufnahme eines Galgens 23 vorgesehen, an dem ein Behältnis für eine Behandlungs-, Kühl- und/ oder Spülflüssigkeit befestigbar ist. Diese Flüssigkeit wird durch eine Fördervorrichtung 6 in Richtung der Behandlungsstelle gefördert. Die Fördervorrichtung 6 ist Teil des medizinischen, insbesondere chirurgischen, Tisch-Steuergeräts 1 und im oder am Außengehäuse 2, insbesondere an einer der Seitenflächen des Außengehäuses 2 angeordnet. Die Fördervorrichtung 6 umfasst insbesondere eine Pumpe, zum Beispiel eine Schlauchquetschpumpe. Die Fördervorrichtung 6, insbesondere die Pumpe, ist vorzugsweise in einer vom übrigen, durch das Außengehäuse 2 gebildeten Innenraum 24 abgetrennten Pumpenkammer 25 vorgesehen, siehe Figur 2.

In den Figur 1 und 3 ist des Weiteren eine am Außengehäuse 2, an der Frontseite 2B, vorgesehene Anschlussvorrichtung 3 an ein mit einer Hand haltbares, medizinisches Behandlungsgerät 4 zu erkennen. Die Anschlussvorrichtung 3 ist vorzugsweise als lösbare Steckverbindung ausgebildet, an die, über einen Versorgungsschlauch oder ein Versorgungskabel 26, das Behandlungsgerät 4 anschließbar ist. Das Behandlungsgerät 4 umfasst insbesondere ein chirurgisches, medizinisches oder dentales Hand- oder Winkelstück, das, vorzugsweise lösbar, mit einem Werkzeug, das auf die Behandlungsstelle einwirkt, verbindbar ist. Ein elektrischer Antrieb des Behandlungsgerät 4 ist vorgesehen, das Werkzeug in Bewegung zu versetzen, zum Beispiel in eine rotierende oder schwingende Bewegung. Über den Versorgungsschlauch 26 wird das Behandlungsgerät 4, insbesondere der elektrische Antrieb, zum Beispiel mit elektrischer Energie und/ oder mit Steuer- oder Regelsignalen versorgt, die insbesondere von einer im Außengehäuse 2 angeordneten Steuervorrichtung 5 erzeugt werden. Über den Versorgungsschlauch 26 können auch drahtgebunden Daten, zum Beispiel Messdaten von Sensoren des Behandlungsgeräts 4, Identifikationsdaten des Behandlungsgeräts 4, Bilddaten einer am Behandlungsgeräts 4 vorgesehenen Kamera, Reinigungs- oder Pflegedaten des Behandlungsgeräts 4 und ähnliche Daten, uni- oder bidirektional übertragen werden, insbesondere an die Steuervorrichtung 5.

Die im Außengehäuse 2 angeordnete Steuervorrichtung 5, siehe Figur 2, ist somit zum Steuern und/ oder Regeln des Tisch-Steuergeräts 1 und/ oder des mit dem Tisch-Steuergerät 1 verbundenen medizinischen Behandlungsgeräts 4 vorgesehen. Sie kann auch mit einer Bildgebungsvorrichtung 9 des Tisch-Steuergeräts 1 Daten übertragend und/ oder Signal übertragend verbunden sein. Die Daten können zum Beispiel Bilddaten umfassen, die von der Bildgebungsvorrichtung 9 generiert werden und anschließend an die Steuervorrichtung 5 übertragen werden. Die Steuervorrichtung 5 kann zum Beispiel ausgebildet sein, die empfangenen Bilddaten zu speichern und/ oder zu be- oder verarbeiten und/ oder an ein Bilddatenverarbeitungssystem 19 des Tisch-Steuergeräts 1 oder an ein externes Bilddatenverarbeitungssystem weiterzuleiten. Die übertragbaren Signale können zum Beispiel Steuersignale zum Ein- oder Ausschalten der Bildgebungsvorrichtung 9, insbesondere der Kamera, und/ oder einer Strahlungsquelle 14 der Bildgebungsvorrichtung 9, umfassen.

Die an der Frontseite 2B vorgesehene Mensch-Maschine-Schnittstelle 7 ist zum Bedienen des Tisch-Steuergeräts 1 und/ oder des mit dem Tisch-Steuergerät 1 verbundenen medizinischen Behandlungsgeräts 4 und/ oder der Bildgebungsvorrichtung 9 und/ oder der Strahlungsquelle 14 vorgesehen. Über die Mensch-Maschine-Schnittstelle 7 sind zum Beispiel Betriebsparameter für das Tisch-Steuergerät 1, das medizinische Behandlungsgerät 4 und/ oder die Bildgebungsvorrichtung 9 eingebbar oder auswählbar. Insbesondere aktiviert der Anwender die Bildgebungsvorrichtung 9 und gegebenenfalls die Strahlungsquelle 14 und/ oder startet die Bilddatenerfassung durch die Bildgebungsvorrichtung 9 über die Mensch-Maschine-Schnittstelle 7, so wie dies im Vorstehenden schon beschrieben ist. Die Mensch-Maschine-Schnittstelle 7 kann dazu Bedienelemente wie Druckknöpfe, Taster und/ oder mechanische Schieber umfassen. Vorzugsweise ist die Mensch-Maschine-Schnittstelle 7 jedoch als Teil der grafischen Anzeigevorrichtung 8 ausgebildet oder durch die grafische Anzeigevorrichtung 8 gebildet, so wie dies in den Figuren 1 und 3 dargestellt ist.

Die grafische Anzeigevorrichtung 8 ist zum Anzeigen von Daten des Tisch-Steuergeräts 1 und/ oder des mit dem Tisch-Steuergerät 1 verbundenen medizinischen Behandlungsgeräts 4 und/ oder der Bildgebungsvorrichtung 9 und/ oder der Strahlungsquelle 14 ausgebildet. Die Daten umfassen zum Beispiel Betriebsdaten und/ oder Messdaten der genannten Geräte und Vorrichtungen 1, 4, 9, 14. Die grafische Anzeigevorrichtung 8 ist insbesondere als Touch-Screen ausgebildet, so dass zumindest ein Teilbereich der grafischen Anzeigevorrichtung 8 auch als Mensch-Maschine-Schnittstelle 7 verwendbar ist, so wie dies im Vorstehenden beschrieben ist.

Das Tisch-Steuergerät 1 umfasst des Weiteren eine Bildgebungsvorrichtung 9, die Bilddaten erzeugt von Materialien und/ oder Gegenständen 10, die bei einer medizinischen Behandlung unter Einsatz des Tisch-Steuergeräts 1 verwendet werden, und/ oder von Identifizierungscodes 11 dieser Materialien oder Gegenstände 10, siehe auch Figur 4. Die Bildgebungsvorrichtung 9 umfasst eine digitale Kamera 29 mit einem digitalen Bildsensor. Die Kamera 29, insbesondere die Kameraoptik und/ oder der Bildsensor, weist eine feststehende oder unveränderliche Brennweite auf.

Im Folgenden sind Merkmale beschrieben, die dem Tisch-Steuergerät 1 der Figuren 1 und 2 eigen sind.

Wie insbesondere aus der Figur 2 zu erkennen ist, ist die Kamera 29 im Innenraum 24 des Außengehäuses 2, nahe oder anschließend an die Frontseite 2B angeordnet. Die Frontseite 2B weist einen Strahlungsdurchlass 12 auf, durch welche Strahlung von außerhalb des Außengehäuses 2 in dieses eintreten können, so dass die Kamera 29 die Strahlung zur Erfassung von Bilddaten empfangen kann. Der Strahlungsdurchlass 12 und die Kamera 29 sind seitlich neben der grafischen Anzeigevorrichtung 8 vorgesehen, ungefähr auf halber Höhe der Frontseite 2B. Die Frontseite 2B ist zum Beispiel aus transparentem Material, insbesondere Kunststoff oder Glas, das an der Oberfläche gefärbt oder beschichtet ist. Der Strahlungsdurchlass 12 ist durch einen Bereich der Frontseite 2B gebildet, der nicht gefärbt oder beschichtet ist. Alternativ ist die Frontseite 2B aus einem farbigen Material, in dem Farbpartikel enthalten sind, und der Strahlungsdurchlass 12 ist durch eine Öffnung gebildet, in der ein transparentes Material aufgenommen ist.

Im Innenraum 24 des Außengehäuses 2 ist des Weiteren eine Strahlungsquelle 14 vorgesehen, die Strahlung mit einem begrenzten Spektralbereich des für ein menschliches Auge sichtbaren Lichtspektrums und/ oder Infrarotstrahlung emittiert. Auch die Strahlungsquelle 14 ist nahe oder anschließend an die Frontseite 2B angeordnet. Das Außengehäuse 2, insbesondere die Frontseite 2B, weist einen Beleuchtungsaustritt 28 auf, durch welche Strahlung der Strahlungsquelle 14 in die Umgebung emittierbar ist, insbesondere in einen Bildaufnahmebereich 30, siehe Figur 4. Der Beleuchtungsaustritt 28 und die Strahlungsquelle 14 sind seitlich neben der grafischen Anzeigevorrichtung 8 vorgesehen, insbesondere auf jener Seite der grafischen Anzeigevorrichtung 8, die der Seite mit dem Strahlungsdurchlass 12 und der Kamera 29 gegenüberliegt. Der Beleuchtungsaustritt 28 und die Strahlungsquelle 14 sind ungefähr auf halber Höhe der Frontseite 2B angeordnet.

Im Folgenden sind Merkmale beschrieben, die dem Tisch-Steuergerät 1 der Figur 3 eigen sind.

Das Tisch-Steuergerät 1 der Figur 3 weist ein Bildgebungsmodul 13 auf, in dem die Bildgebungsvorrichtung 9 angeordnet ist, wobei das Bildgebungsmodul 13 außerhalb des Außengehäuses 2 des Tisch-Steuergeräts 1 ausgebildet oder vorgesehen ist. Das Bildgebungsmodul 13 ist somit ein eigenständiges, separates Element. Ein, vorzugsweise quader- oder würfelförmiges, Modulgehäuse 31 grenzt das Bildgebungsmodul 13 zur Umgebung ab. Im Inneren des Bildgebungsmoduls 13 oder des Modulgehäuses 31 ist die digitale Kamera 29 mit dem digitalen Bildsensor der Bildgebungsvorrichtung 9 angeordnet.

Das Modulgehäuse 31 weist einen Strahlungsdurchlass 12 auf, durch welche die Kamera 29 Strahlung von den verwendeten Materialien, Gegenständen 10 und/ oder deren Identifizierungscodes 11 empfängt. Im Innenraum des Modulgehäuses 31 ist des Weiteren eine Strahlungsquelle 14 vorgesehen, die Strahlung mit einem begrenzten Spektralbereich des für ein menschliches Auge sichtbaren Lichtspektrums und/ oder Infrarotstrahlung emittiert.

Das Modulgehäuse 31 weist einen Beleuchtungsaustritt 28 auf, durch welche Strahlung der Strahlungsquelle 14 in die Umgebung emittierbar ist, insbesondere in den Bildaufnahmebereich 30. Der Strahlungsdurchlass 12 und der Beleuchtungsaustritt 28 sind vorzugsweise an derselben Wand oder Frontseite 31A des Modulgehäuses 31 vorgesehen.

Im Folgenden sind wiederum Merkmale beschrieben, welche die Tisch-Steuergeräte 1 der Figur 1 und der Figur 3 gemein haben.

Die Strahlungsquelle 14 umfasst ein optisches Halbleiterelement, insbesondere eine Leuchtdiode. Die Strahlungsquelle 14 emittiert grünes Licht, i.e. Strahlung mit einer Wellenlänge im Bereich von etwa 500 nm - 570 nm, vorzugsweise mit einem Peak bei 525 nm, zur Beleuchtung der Identifizierungscodes 11, der Materialien und/ oder Gegenstände 10 während der Erfassung der Bilddaten.

Die Bildgebungsvorrichtung 9, insbesondere die Kamera 29, weist einen optischen Filter 15 auf (nur in der Figur 2 dargestellt), der das von den Identifizierungscodes 11, Materialien und/ oder Gegenständen 10 einfallende Strahlung filtert. Der optische Filter 15 ist in etwa oder ausschließlich für Wellenlängen der von der Strahlungsquelle 14 emittierten Strahlung durchlässig, insbesondere für grünes Licht. Der optische Filter 15 ist an der oder anschließend an den Strahlungsdurchlass 12 der Frontseite 2B oder 31A angeordnet, so dass das von den Materialien, Gegenständen 10 und/ oder Identifizierungscodes 11 empfangene Strahlung vor dem Auftreffen auf dem Bildsensor der Kamera 29 den optischen Filter 15 passiert.

Aus der Figur 2 ist zu erkennen, dass die Bildgebungsvorrichtung 9, insbesondere die Kamera 29, und die Strahlungsquelle 14, schräg zur Frontseite 2B und/ oder zur grafischen Anzeigevorrichtung 8, insbesondere zu deren vom Innenraum 24 abgewandten, äußeren Bildschirmoberfläche, angeordnet ist/ sind. Entsprechend ist auch die Bildgebungsvorrichtung 9, insbesondere die Kamera 29, und die Strahlungsquelle 14, des Bildgebungsmoduls 13 schräg zur Frontseite 31A angeordnet. Die Kamera 29 und die Strahlungsquelle 14 sind dabei zueinander gedreht positioniert.

In der Figur 4 ist zu erkennen, dass ein Sichtfeld 16 oder eine optische Achse 16A der Kamera 29 in einem Winkel ungleich 90° zur Frontseite 2B des Tisch-Steuergeräts 1 oder zur Frontseite 31A des Bildgebungsmoduls 13 angeordnet ist. Auch eine Längsachse 18A der Strahlungsquelle 14 oder eine Kegelachse 18A eines abgestrahlten Strahlungskegels 18 der Strahlungsquelle 14 ist in einem Winkel ungleich 90° zu der Frontseite 2B oder 31A angeordnet. Die Kamera 29 und die Strahlungsquelle 14 sind derart angeordnet oder einander zugewandt, dass die optische Achse 16A und Achse 18A sich in einen Bildaufnahmebereich 30 erstrecken, insbesondere einander in dem Bildaufnahmebereich 30 treffen oder schneiden. Der Bildaufnahmebereich 30 ist ein Raumvolumen, in welches zu erfassende Identifizierungscodes 11 und/ oder Materialien oder Gegenstände 10 eingebracht werden müssen, damit in den von der Bildgebungsvorrichtung 9 erzeugten Bilddaten Informationen enthalten sind, die für ein Bilddatenverarbeitungssystem 19 auswertbar sind und/ oder eine Zeichenerkennung ermöglichen. Der Bildaufnahmebereich 30, insbesondere die Entfernung des Bildaufnahmebereichs 30 von dem Bildsensor und/ oder dem Strahlungsdurchlass 12, ist somit auf die unveränderbare Brennweite der Kamera 29 abgestimmt.

In dem Außengehäuse 2 des Tisch-Steuergeräts 1 oder in dem Bildgebungsmodul 13 ist des Weiteren ein Bilddatenverarbeitungssystem 19 angeordnet, das zur Verarbeitung der von der Bildgebungsvorrichtung 9 erfassten und/ oder zur Verfügung gestellten Bilddaten ausgebildet ist. Das Bilddatenverarbeitungssystem 19 ist kommunikativ mit der Kamera 29 verbunden, um Bilddaten des Bildsensors zu empfangen. Das Bilddatenverarbeitungssystem 19 umfasst einen Prozessor mit Bildverarbeitungssoftware, der die erfassten Bilddaten verarbeitet und/ oder eine Zeichenerkennung durchführt, so wie dies im Vorstehenden beschrieben ist.

Das Tisch-Steuergerät 1 und das Bildgebungsmodul 13 umfasst eine Signalvorrichtung 20, die operativ mit dem Bilddatenverarbeitungssystem 19 verbunden ist. Die Signalvorrichtung 20 ist ausgebildet, ein für den Anwender wahrnehmbares Signal abzugeben, wenn das Bilddatenverarbeitungssystem 19 erkennt, dass zumindest eine optische Eigenschaft, zum Beispiel der Kontrast, die Bildschärfe und/ oder Auflösung, der erfassten Bilddaten einen vorbestimmten Grenzwert erreicht, und/ oder wenn das Bilddatenverarbeitungssystem 19 einen Code und/ oder ein Symbol und/ oder ein alphanumerisches Zeichen in den von der Bildgebungsvorrichtung 9 erfassten Bilddaten erkennt. Die Signalvorrichtung 20 des Tisch-Steuergeräts 1 der Figuren 1 und 2 ist beispielhaft ausgebildet, ein akustisches Signal zu erzeugen, während die Signalvorrichtung 20 des Bildgebungsmodul 13 ein visuelles Signal abgibt und außen auf dem Modulgehäuse 31 angeordnet ist, um für den Anwender sichtbar zu sein.

Das Tisch-Steuergerät 1 und das Bildgebungsmodul 13 umfasst eine Kommunikationsvorrichtung 21 zur Übertragung 17 der von der Bildgebungsvorrichtung 9 erfassten Bilddaten an eine externe Datenverarbeitungsvorrichtung 22. Die Datenverarbeitungsvorrichtung 22, die zum Beispiel auch in der Cloud vorgesehen sein kann, ist ausgebildet, die Bilddaten zu bearbeiten, speichern, und/ oder mit weiteren Daten, zum Beispiel Patientendaten, zu verbinden.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar.

## Patentansprüche

1. Medizinisches, insbesondere chirurgisches, Tisch-Steuergerät (1), umfassend:
ein Außengehäuse (2), welches das Tisch-Steuergerät (1) zur Umgebung abgrenzt,
eine am Außengehäuse (2) vorgesehene Anschlussvorrichtung (3) an ein mit einer Hand haltbares, medizinisches Behandlungsgerät (4),
eine im Außengehäuse (2) angeordnete Steuervorrichtung (5) zum Steuern des Tisch-Steuergeräts (1) und/ oder des mit dem Tisch-Steuergerät (1) verbundenen medizinischen Behandlungsgeräts (4),
eine im oder am Außengehäuse (2) angeordnete Fördervorrichtung (6) zum Fördern einer Behandlungs- und/ oder Kühlflüssigkeit, und
eine Mensch-Maschine-Schnittstelle (7, 8) zum Bedienen des Tisch-Steuergeräts (1) und/ oder des mit dem Tisch-Steuergerät (1) verbundenen medizinischen Behandlungsgeräts (4), **gekennzeichnet durch**
eine Bildgebungsvorrichtung (9), die Bilddaten von Materialien und/ oder Gegenständen (10) und/ oder von Identifizierungscodes (11) dieser Materialien oder Gegenstände (10) erzeugt, die bei einer medizinischen Behandlung verwendet werden, bei der das Tisch-Steuergerät (1) eingesetzt wird.

2. Medizinisches, insbesondere chirurgisches, Tisch-Steuergerät (1) nach Anspruch 1, **dadurch gekennzeichnet, dass**
die Bildgebungsvorrichtung (9) im Außengehäuse (2) angeordnet ist und das Außengehäuse (2) einen Strahlungsdurchlass (12) aufweist, durch welche die Bildgebungsvorrichtung (9) Strahlung empfängt.

3. Medizinisches, insbesondere chirurgisches, Tisch-Steuergerät (1) nach Anspruch 1, **gekennzeichnet durch**
ein Bildgebungsmodul (13), in dem die Bildgebungsvorrichtung (9) angeordnet ist, wobei das Bildgebungsmodul (13) außerhalb des Außengehäuses (2) des Tisch-Steuergeräts (1) ausgebildet ist.

4. Medizinisches, insbesondere chirurgisches, Tisch-Steuergerät (1) nach einem der vorstehenden Ansprüche, **gekennzeichnet durch**
eine Strahlungsquelle (14), die Strahlung mit einem begrenzten Spektralbereich des für ein menschliches Auge sichtbaren Lichtspektrums und/ oder Infrarotstrahlung emittiert.

5. Medizinisches, insbesondere chirurgisches, Tisch-Steuergerät (1) nach Anspruch 4, **dadurch gekennzeichnet, dass**
die Strahlungsquelle (14) grüne Strahlung abstrahlt.

6. Medizinisches, insbesondere chirurgisches, Tisch-Steuergerät (1) nach Anspruch 4 oder 5, **dadurch gekennzeichnet, dass**
die Strahlungsquelle (14) durch eine Leuchtdiode und/ oder durch eine grafische Anzeigevorrichtung (8) des Tisch-Steuergeräts gebildet ist.

7. Medizinisches, insbesondere chirurgisches, Tisch-Steuergerät (1) nach Anspruch 4, 5 oder 6, **dadurch gekennzeichnet, dass**
die Bildgebungsvorrichtung (9) einen optischen Filter (15) aufweist, der im Wesentlichen nur für Wellenlängen durchlässig ist, die in der von der Strahlungsquelle (14) emittierte Strahlung enthalten sind.

8. Medizinisches, insbesondere chirurgisches, Tisch-Steuergerät (1) nach einem der vorstehenden Ansprüche, **dadurch gekennzeichnet, dass**
die Bildgebungsvorrichtung (9), insbesondere eine optische Achse (16A) einer Kamera (29) der Bildgebungsvorrichtung (9), in einem Winkel ungleich 90° zu einer Frontseite (2B) des Tisch-Steuergeräts (1) oder des Bildgebungsmoduls (13) angeordnet ist.

9. Medizinisches, insbesondere chirurgisches, Tisch-Steuergerät (1) nach einem der Ansprüche 4 - 8, **dadurch gekennzeichnet, dass**
die Strahlungsquelle (14), insbesondere eine Längsachse oder Kegelachse (18A) eines abgestrahlten Strahlungskegels (18) der Strahlungsquelle (14), in einem Winkel ungleich 90° zu einer Frontseite (2B) des Tisch-Steuergeräts (1) oder des Bildgebungsmoduls (13) angeordnet ist.

10. Medizinisches, insbesondere chirurgisches, Tisch-Steuergerät (1) nach Anspruch 9, **dadurch gekennzeichnet, dass**
die Bildgebungsvorrichtung (9) und die Strahlungsquelle (14) derart angeordnet sind, dass ein Sichtfeld (16), insbesondere die optische Achse (16A), der Bildgebungsvorrichtung (9) und der von der Strahlungsquelle (14) abgestrahlte Strahlungskegel (18), insbesondere die Kegelachse (18A) des Strahlungskegels (18), einander schneiden.

11. Medizinisches, insbesondere chirurgisches, Tisch-Steuergerät (1) nach einem der vorstehenden Ansprüche, **gekennzeichnet durch**
eine im Außengehäuse (2) des Tisch-Steuergeräts (1) oder in dem Bildgebungsmodul (13) angeordnete Bilddatenverarbeitungssystem (19) zur Verarbeitung der von der Bildgebungsvorrichtung (9) erfassten Bilddaten.

12. Medizinisches, insbesondere chirurgisches, Tisch-Steuergerät (1) nach Anspruch 11, **dadurch gekennzeichnet, dass**
das Bilddatenverarbeitungssystem (19) zur optischen Zeichenerkennung von Codes und/ oder Symbolen und/ oder alphanumerischen Zeichen, die in den von der Bildgebungsvorrichtung (9) erfassten Bilddaten enthalten sind, ausgebildet ist.

13. Medizinisches, insbesondere chirurgisches, Tisch-Steuergerät (1) nach Anspruch 11 oder 12, **gekennzeichnet durch**
eine Signalvorrichtung (20), die operativ mit dem Bilddatenverarbeitungssystem (19) verbunden und derart ausgebildet ist, dass, wenn das Bilddatenverarbeitungssystem (19) erkennt, dass zumindest eine optische Eigenschaft, zum Beispiel der Kontrast, die Bildschärfe und/ oder Auflösung, der erfassten Bilddaten einen vorbestimmten Grenzwert erreicht, und/ oder wenn das Bilddatenverarbeitungssystem (19) einen Code und/ oder ein Symbol und/ oder ein alphanumerisches Zeichen in den von der Bildgebungsvorrichtung (9) erfassten Bilddaten erkennt, ein für den Anwender wahrnehmbares Signal abgibt.

14. Medizinisches, insbesondere chirurgisches, Tisch-Steuergerät (1) nach einem der vorstehenden Ansprüche, **gekennzeichnet durch**
eine Kommunikationsvorrichtung (21) zur Übertragung der von der Bildgebungsvorrichtung (9) erfassten Bilddaten an eine externe Datenverarbeitungsvorrichtung (22).

15. Verfahren zur Registrierung von Materialien und/ oder Gegenständen (10), die bei einer medizinischen Behandlung verwendet werden, bei der ein medizinisches, insbesondere chirurgisches, Tisch-Steuergerät (1) eingesetzt wird, **dadurch gekennzeichnet, dass**
ein medizinisches, insbesondere chirurgisches, Tisch-Steuergerät (1) mit einer Bildgebungsvorrichtung (9), vorzugsweise ein medizinisches, insbesondere chirurgisches, Tisch-Steuergerät (1) nach einem der vorstehenden Ansprüche 1 - 14 zur Verfügung gestellt wird, und
dass Bilddaten von Materialien und/ oder Gegenständen (10), die bei der medizinischen Behandlung verwendet werden, und/ oder von Identifizierungscodes (11) dieser Materialien oder Gegenstände (10) von der Bildgebungsvorrichtung (9) des Tisch-Steuergeräts (1) erzeugt werden.
